# EUROPEAN PATENT APPLICATION

(11) **EP 4 218 868 A1**
(43) Date of publication of application: **02.08.2023**
(21) Application number: 21884635.0
(22) Date of filing: 27.08.2021
(51) Int. Cl.: A61M 15/06, A61M 15/00, A61M 11/00, A24F 47/00, A24F 40/48, A24F 40/40, A24F 40/10

(54) **ATOMIZER HAVING INTEGRATED ATOMIZATION ASSEMBLY**

(30) Priority: 27.10.2020 CN 202011167148
(71) Applicant: Huizhou Happy Vaping Technology Limited, Huizhou, Guangdong 516000 (CN)
(72) Inventor: LIN, Guangrong, Guangdong 516000 (CN); ZHENG, Xianbin, Guangdong 516000 (CN)
(74) Representative: Meyer, Thorsten
(86) International application number: PCT/CN2021/114963
(87) International publication number: WO 2022/088903

(57) **Abstract**

The disclosure provides a vaporizer having an integrated vaporizing assembly, which comprises a housing having a mouthpiece end and a connecting end, and an integrated vaporizing assembly. The mouthpiece end is provided with a mouthpiece. The connecting end is provided with an opening. The housing may be connected with a battery part by the connecting end, to form an electronic vaporizing device. The integrated vaporizing assembly is disposed into the housing via the opening. The integrated vaporizing assembly comprises, from top to bottom, a vaporizing core base, a vaporizing core, and a base part connected with the vaporizing core base. The lower portion of the vaporizing core base is provided with a cavity concaving upward, in which the vaporizing core is mounted. The vaporizing core comprises a vaporizing core body for absorbing the to-be-vaporized liquid and a heating resistance for heating the to-be-vaporized liquid. The vaporizing core body is provided with a vaporizing passage. An air chamber in communication with the vaporizing passage is further provided between the vaporizing core and the base part. The base part closes the opening of the housing. It has advantages of stable vaporizing process and optimal vaporizing effect during the heating and vaporizing process. It would not be charred to produce a burning taste. It has a firm structure which is not easy to deform, avoiding liquid leakage and air leakage.

## Description

### TECHNICAL FIELD

The disclosure relates to the technical field of electronic vaporizing device, and more particularly, to a vaporizer having an integrated vaporizing assembly.

### BACKGROUND

Usually an electronic vaporizing device includes a battery part and a vaporizer part. A battery cell inside the battery part is to supply power to the vaporizer part. The vaporizer part includes a heating unit which can atomize the to-be-vaporized solution into vapor for users to vape when powered on.

In particular, for electronic vaporizing devices, such as the ones which are used as medicine vaporizing devices, the basic task is to provide a vaporizing process for converting the solutions, such as to-be-vaporized liquid or medicine liquid, stored in the electronic vaporizing device into vapor fog, aerosol, steam, etc.

Existing vaporizers of the electronic vaporizing device usually use a liquid guiding wick which is formed by elongated glass cords or cotton strings. Heating coils for the heating process are directly wound around the glass cords or cotton strings. However, during the heating process, the liquid guiding wick which has a small size and thus poor liquid storage performance may be charred due to low liquid caused by insufficient transmission of the to-be-vaporized solution, thereby producing a burning taste and bringing bad user experience. Furthermore, the vaporizers, which use a liquid guiding wick formed by glass cords or cotton strings, usually have a structure in which the glass cords or cotton strings are transversely arranged above the vaporizing base, with two ends of the glass cords or cotton strings extending into the liquid storage chamber to absorb and transmit the to-be-vaporized liquid. Such structure fails to provide a tight seal between the liquid storage chamber and the glass cords or cotton strings. Thus, the leakage may occur when a greater suction is applied. Furthermore, in order to facilitate assembly during the manufacture process, it needs to wind the glass cords or cotton strings around the heating coils, then mount them on the vaporizing base, and then dispose the vaporizing base and other components into the vaporizer housing in turn. Hence, it has complicated structure, inconvenient assembly, low production efficiency, and low degree of automatic production.

### SUMMARY

### Technical problem

An object of the disclosure is to overcome the above shortcomings and provide a vaporizer having an integrated vaporizing assembly.

### Technical solutions

The disclosure provides a technical solution as follow. A vaporizer having an integrated vaporizing assembly comprises a housing having a mouthpiece end and a connecting end. The mouthpiece end is provided with a mouthpiece. The connecting end is provided with an opening. The housing may be connected with a battery part by the connecting end, to form an electronic vaporizing device. The integrated vaporizing assembly is further provided. The integrated vaporizing assembly may be disposed into the housing via the opening of the connecting end. The integrated vaporizing assembly is provided with, from top to bottom, a vaporizing core base, a vaporizing core, and a base part, wherein the vaporizing core base is connected with the base part. The lower portion of the vaporizing core base is provided with a cavity which is concave upward, and the vaporizing core is mounted in the cavity. The vaporizing core comprises a vaporizing core body for transmission of the to-be-vaporized liquid and a heating resistance for heating the to-be-vaporized liquid. The vaporizing core body is provided with a vaporizing passage. An air chamber in communication with the vaporizing passage is further provided between the vaporizing core and the base part. The base part closes the opening of the housing.

Preferably, a vapor fog through-hole which is in communication with the mouthpiece and the vaporizing passage may be provided inside the vaporizing core base.

Preferably, the vapor fog through-hole may be centrally formed in the vaporizing core base.

Preferably, the space between the vaporizing core base and the inner wall of the housing may define a liquid storage chamber for storing the to-be-vaporized liquid. The vaporizing core base may be internally provided with a liquid through-hole for communicating the liquid storage chamber with the vaporizing core.

Preferably, the upper portion of vaporizing core body may be provided with a liquid guiding groove which is concave downward and in communication with the liquid through-hole.

Preferably, the vaporizing passage of the vaporizing core body may be defined by the central through-hole centrally formed in the vaporizing core body or by vertical grooves provided on two sides of the vaporizing core body.

Preferably, the mouthpiece may extend inwards and may be integrally formed with a vapor outlet tube. The vaporizing core base may comprise an upper section, a middle section, and a lower section, which have different outer diameters, wherein the upper section of the vaporizing core base is formed as a tube-like connector for connecting with the lower end of the vapor outlet tube, the outer wall of the middle section of the vaporizing core base is sheathed in the inner wall of the housing, the outer wall of the lower section of the vaporizing core base is connected to the inner wall of the upper portion of the base part.

Preferably, the vaporizing core base may be formed by a support portion and a sealing portion which are embedded with each other, wherein the sealing portion comprises the tube-like connector, the outer wall of the middle section of the vaporizing core base, and the inner wall of the cavity of the vaporizing core base, the support portion is made of hard material, and the sealing portion is made of soft material.

Preferably, first engaging holes may be provided on two sides of the connecting end, and the side wall of the base part may be provided with first snap bumps for engaging with the first engaging holes.

Preferably, the outer wall of the lower portion of the vaporizing core base may be provided with second snap bumps, two ends of the base part may be arranged with protruding columns, respectively, and the protruding columns may be provided with second engaging holes for engaging with the second snap bumps.

Preferably, inside the base part, supporting parts for propping up the vaporizing core body may be provided.

Preferably, the supporting parts may include two positive and negative electrode columns served as positive and negative electrodes, and the positive and negative electrode columns may be respectively connected with two ends of the heating resistance through contact.

Preferably, the wall portion of the connecting end may be provided with a main air inlet in communication with the air chamber.

Preferably, the base part may be provided with an air through-hole in communication with the air chamber.

Preferably, the air through-hole may be centrally formed in the base part or defined on two sides of the base part, and the upper portion of the air through-hole may be defined by a tubular part and protrudes from the inner bottom surface of the base part.

Preferably, an inclined cover may be placed on the top of the tubular part, wherein the inclined cover may be provided with an air inlet aperture, or one end of the inclined cover is placed higher than the wall portion of the tubular part to expose an air entrance.

Preferably, the vaporizing core body may be made of microporous ceramic material or microporous diatomaceous material.

Preferably, the heating resistance may be arranged on the lower surface of the vaporizing core body or on the surface of the vaporizing passage.

Preferably, the heating resistance may comprise two planar contact terminals respectively electrically connected with positive and negative electrode columns of the base part through contact.

Preferably, the outer surface of the base part may be embedded with magnets or magnetic materials, and the magnets or magnetic materials and another set of magnets or magnetic materials provided on the battery part may have a mutual magnetic attraction force for magnetically connecting the vaporizer with the battery part.

### Advantages

Instead of liquid guiding materials such as the glass cords or cotton strings, the vaporizing core body made of microporous materials is provided for transmission of the to-be-vaporized liquid in the disclosure. It has good liquid storage performance and transmission performance, thereby achieving stable vaporizing process and optimal vaporizing effect. Furthermore, it has good heat resistance and would not be charred to produce a burning taste during the heating and vaporizing process. According to the structure of the vaporizer in the disclosure, as the vaporizing core is placed upside-down and mounted in the vaporizing core base which is formed by the support portion and the sealing portion embedded with each other, it facilitates quick transmission of the to-be-vaporized liquid. It has a firm structure with good sealing effect, avoiding liquid leakage and air leakage. In addition, the disclosure provides the integrated vaporizing assembly, facilitating a simple structure, convenient assembly, greatly improved production efficiency, and high degree of full automatic production.

### BRIEF DESCRIPTION OF THE DRAWINGS

FIG. 1 is a perspective structural view illustrating a vaporizer according to the disclosure in a detached state;
FIG.2 is a front cross-sectional view 1 of a vaporizer according to the disclosure;
FIG.3 is a front cross-sectional view of a vaporizer housing according to the disclosure;
FIG.4 is a perspective exploded view of an integrated vaporizing assembly according to the disclosure;
FIG. 5 is a front view of a vaporizing core base according to the disclosure;
FIG.6 is a top view of a vaporizing core base according to the disclosure;
FIG.7 is a front cross-sectional view of a vaporizing core base according to the disclosure;
FIG.8 is a side cross-sectional view of a vaporizing core base according to the disclosure;
FIG.9 is a perspective structural view illustrating a vaporizing core base according to the disclosure in a detached state;
FIG. 10 is a front view of a vaporizing core according to the disclosure;
FIG. 11 is a top view of a vaporizing core according to the disclosure;
FIG. 12 is a bottom view of a vaporizing core according to the disclosure;
FIG. 13 is a side view of a vaporizing core according to the disclosure;
FIG. 14 is a front cross-sectional view of a vaporizing core according to the disclosure;
FIG. 15 is a side cross-sectional view of a vaporizing core according to the disclosure;
FIG. 16 is a perspective view 1 of a base part according to the disclosure;
FIG. 17 is a perspective view 2 of a base part according to the disclosure;
FIG. 18 is a perspective view 3 of a base part according to the disclosure;
FIG. 19 is a front cross-sectional view of a base part according to the disclosure;
FIG.20 is a front cross-sectional view 2 of a vaporizer according to the disclosure;
FIG.21 is a side cross-sectional view of a vaporizer according to the disclosure;
FIG.22 is a perspective view of a base part according to another embodiment of the disclosure;
FIG.23 is a perspective view 1 of a base part according to yet another embodiment of the disclosure;
FIG.24 is a perspective view 2 of a base part according to yet another embodiment of the disclosure;
FIG.25 is a perspective view of a base part according to yet another embodiment of the disclosure;
FIG.26 is a front cross-sectional view of a base part according to yet another embodiment of the disclosure;
FIG.27 is a side cross-sectional view of a base part according to yet another embodiment of the disclosure;
FIG.28 is a bottom view of a vaporizing core according to yet another embodiment of the disclosure;
FIG.29 is a front cross-sectional view of a vaporizing core according to yet another embodiment of the disclosure;
FIG.30 is a top view of a vaporizing core according to yet another embodiment of the disclosure;
FIG.31 is a bottom view of a vaporizing core according to yet another embodiment of the disclosure.

### DETAILED DESCRIPTION OF ILLUSTRATED EMBODIMENTS

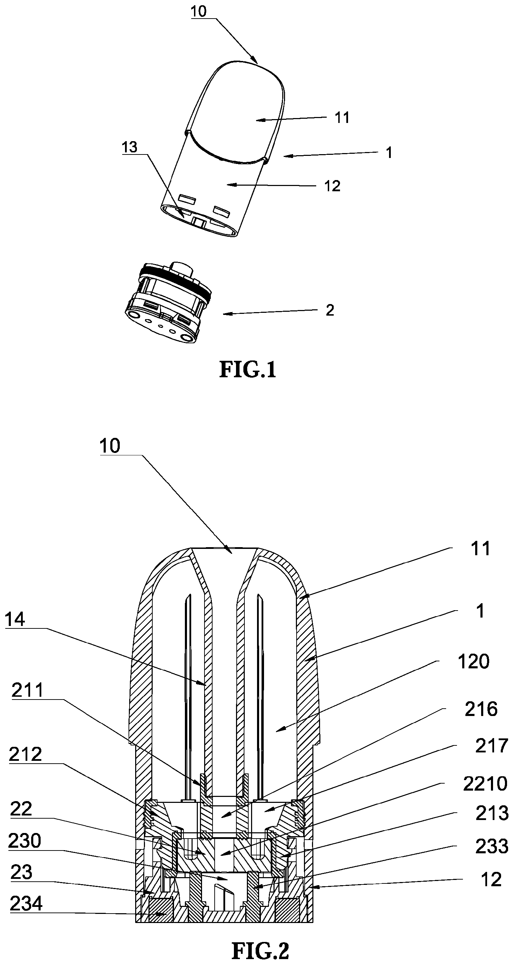

For convenience of description, the vaporizer having an integrated vaporizing assembly according to the disclosure is described in a state in which the vaporizer is vertically disposed, with the mouthpiece 10 of the vaporizer housing facing upwards, as shown in FIG.2. It should be noted that the terms, such as "up", "down", "upper portion", "lower portion", "top end", "bottom end", "top side", and "bottom side" as used in the description, refer to position and orientation relationships of the components of the vaporizer which is vertically disposed in such a manner that the mouthpiece faces upwards.

### Embodiments

Referring to FIGs.1-3, a vaporizer having an integrated vaporizing assembly according to the disclosure mainly consists of a housing 1 and an integrated vaporizing assembly 2. The housing 1 has an upper end and a lower end, namely a mouthpiece end 11 and a connecting end 12. The mouthpiece end 11 is provided with a mouthpiece 10. The mouthpiece 10 extends inwards and is integrally formed with a vapor outlet tube 14. A portion of the vapor outlet tube 14 which defines the mouthpiece 10 has a flared horn-like shape. The connecting end 12 is provided with an opening 13. The housing 1 may be connected with a battery part (not shown in the drawings) by the connecting end 12, to form an electronic vaporizing device. Inside the housing 1, the integrated vaporizing assembly 2 is located. The integrated vaporizing assembly 2 may be disposed into the housing 1 via the opening 13. It is convenient to assemble the integrated vaporizing assembly 2 first, and then dispose it into the housing 1. Thus, it has reliable mounting with good quality and improved production efficiency, and it facilitates automatic production.

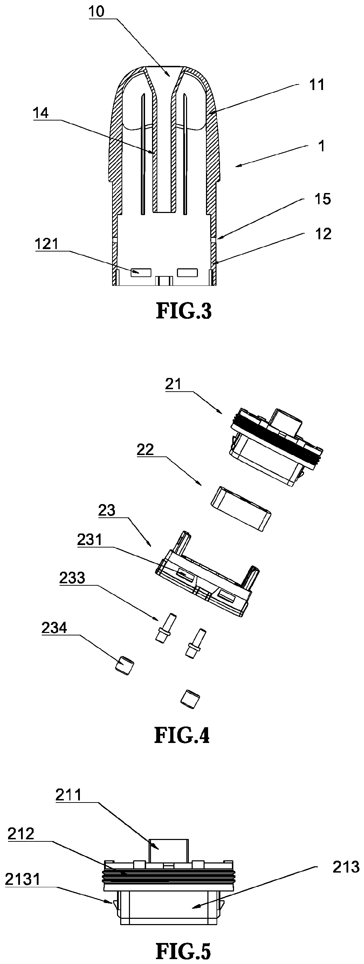

Referring to FIG.4, the integrated vaporizing assembly 2 is provided with, from top to bottom, a vaporizing core base 21, a vaporizing core 22, and a base part 23, wherein the vaporizing core base 21 is connected with the base part 23.

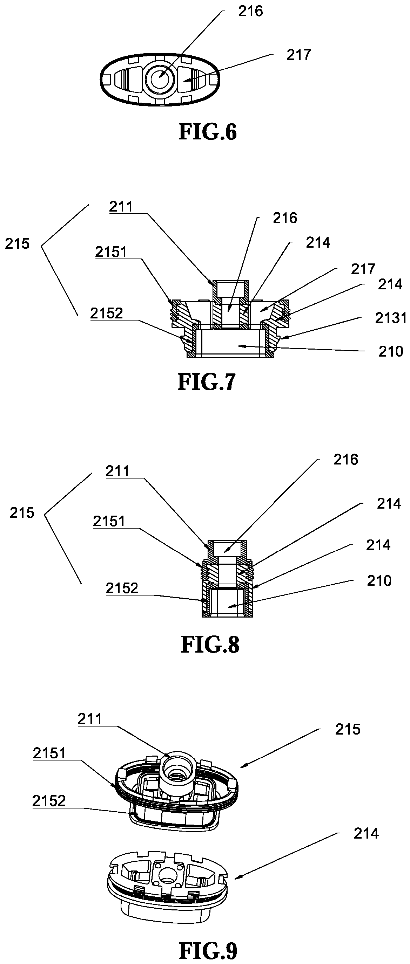

Referring to FIGs.7 and 8, a lower portion of the vaporizing core base 21 is centrally formed with a cavity 210 extending upwards, and the vaporizing core 22 is mounted in the cavity 210. Compared with the traditional arrangement that liquid guiding materials such as the glass cords or cotton strings are transversely arranged above the vaporizing base, the arrangement that the vaporizing core 22 is placed upside-down and mounted in the cavity 210 at the lower end of the vaporizing core base 21 can facilitate downward transmission of the to-be-vaporized liquid directly from the overlying liquid storage chamber to the underlying vaporizing core 22. It achieves high efficiency of liquid transmission and thus stable vaporizing process and optimal vaporizing effect.

Referring to FIG.5, the vaporizing core base 21 comprises an upper section 211, a middle section 212, and a lower section 213, which have different outer diameters. Herein, the upper section 211 of the vaporizing core base 21 is formed as a tube-like connector 211 for connecting with the lower end of the vapor outlet tube 14. The outer wall of the middle section 212 of the vaporizing core base 21 is sheathed in the inner wall of the connecting end 12 of the housing. The outer wall of the lower section 213 of the vaporizing core base 21 is connected to the inner wall of the upper portion of the base part 23.

Referring to FIGs.7-9, the vaporizing core base 21 is formed by a support portion 214 and a sealing portion 215 which are integrally formed and embedded with each other. The sealing portion 215 includes the tube-like connector 211, a portion 2151 of the outer wall of the middle section of the vaporizing core base, and the inner wall 2152 of the cavity of the vaporizing core base. The sealing portion 215 is made of soft material having sealing performance. In such a manner, the upper section 211 of the vaporizing core base 21 can be sealingly connected with the vapor outlet tube 14, the outer wall of the middle section 212 can be sealingly connected with the inner wall of the housing 1, the inner wall 2152 of the cavity 210 of the vaporizing core base can be sealingly connected with the vaporizing core body 221. Thus, it ensures that the liquid storage chamber 120 is sealed to avoid leakage. It ensures a liquid-tight and air-tight seal between a vapor fog through-hole 216 mentioned below with both the liquid storage chamber 120 and the liquid through-hole 217. Further, it ensures a seal between the vaporizing core base 21 and the vaporizing core 22 and prevents leakage of the liquid into an air chamber 230. The support portion 214 is made of hard material, such that the vaporizing core base 21 may have a certain strength and rigidity to make sure that the vaporizing core base 21 is prevented from being deformed to cause loose or leakage.

Referring to FIGs.2, 7, and 8, the vaporizing core base 21 is internally provided with a vapor fog through-hole 216 for communicating the mouthpiece 10 with a vaporizing passage 2210. The vapor fog through-hole 216 is centrally formed in the vaporizing core base 21.

Referring to FIGs.2, 6, and 7, the space between the vaporizing core base 21 and the inner wall of the housing 1 defines the liquid storage chamber 120 for storing the to-be-vaporized liquid. The vaporizing core base 21 is internally provided with a liquid through-hole 217 for communicating liquid storage chamber 120 with the vaporizing core 22.

Referring to FIGs.2, 3, 20, and 21, an air chamber 230 in communication with the vaporizing passage 2210 of the vaporizing core 22 is further provided between the vaporizing core 22 and the base part 23. The base part 23 closes the opening 13 of the housing 1. The wall portion of the connecting end 12 of the housing 1 is provided with a main air inlet 15 in communication with the air chamber 230. External air can pass through the main air inlet 15, then the gap between the vaporizing core base 21 and the base part 23, and then enter the air chamber 230.

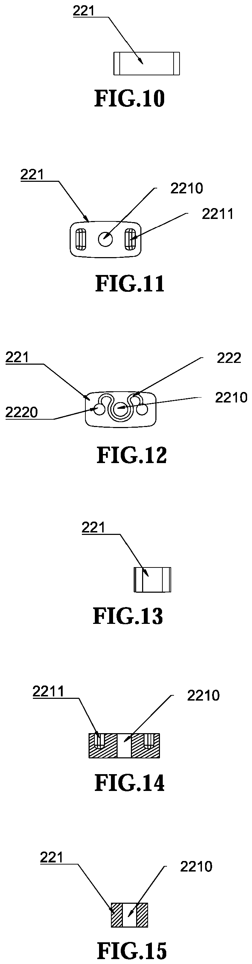

Referring to FIGs. 10-15, the vaporizing core 22 comprises a vaporizing core body 221 made of cellular material and serving for transmission of the to-be-vaporized liquid, and comprises a heating resistance 222 for heating the to-be-vaporized liquid. The vaporizing core body 221 is made of cellular material, in particular microporous ceramic material and microporous diatomaceous material, through which the to-be-vaporized liquid can be easily permeated and transmitted. The vaporizing core body 221 is provided with a vaporizing passage 2210. The vaporizing passage 2210 is defined by a central through-hole 2210 centrally formed in the vaporizing core body. The vaporized vapor fog or aerosol may pass through the vaporizing passage 2210. The upper portion of vaporizing core body 221 is provided with a liquid guiding groove 2211 which is concave downward and in communication with the liquid through-hole 217. The liquid guiding groove 2211, which is formed as a groove concaving downward, serves to store and transmit the to-be-vaporized liquid. Due to the liquid guiding groove 2211, the contact area between the to-be-vaporized liquid and the vaporizing core body 221 can be increased, facilitating quick transmission of the to-be-vaporized liquid into the vaporizing core body, improving liquid transmission performance, and thus achieving optimal vaporizing effect. The heating resistance 222 is placed on the bottom surface of the vaporizing core body 221. The heating resistance 222 is arranged at two ends with planar contact terminals 2220. The two planar contact terminals 2220 are respectively electrically connected with positive and negative electrode columns 233 of the base part 23 through contact.

Referring to FIGs.3 and 4, first engaging holes 121 are provided on two sides of the connecting end 12 of the housing 1, and the side wall of the base part 23 is provided with first snap bumps 231 for engaging with the first engaging holes 121. Thus, with respective engagement of the first snap bumps 231 and the first engaging holes 121, the base part 23 can be conveniently fixedly connected with the housing 1 in an undetachable manner.

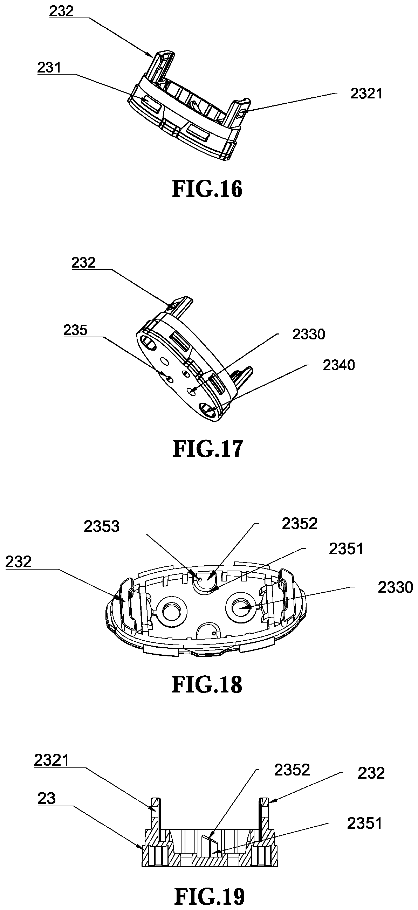

Referring to FIGs.5, 7, and 16, the outer wall of the lower portion of the vaporizing core base 21 is provided with second snap bumps 2131, two ends of the base part 23 are respectively arranged with protruding columns 232, and the protruding columns 232 are provided with second engaging holes 2321 for engaging with the second snap bumps 2131. Thus, with respective engagement of the second snap bumps 2131 and the second engaging holes 2321, the vaporizing core base 21 can be conveniently fixedly connected with the base part 23 in an undetachable manner.

Referring to FIGs.2, 4, 17, and 18, inside the base part 23, supporting parts 233 for propping up the vaporizing core body 221 are provided. The supporting parts 233 function to prop up and fix the vaporizing core body 221 in the cavity 210 of the vaporizing core base 21, and meanwhile ensure that a space for the air chamber 230 is reserved in the base part 23. In the embodiment, the supporting parts include the two positive and negative electrode columns 233 served as positive and negative electrodes. The positive and negative electrode columns 233 are respectively connected with planar contact terminals 2220 at two ends of the heating resistance through contact. The positive and negative electrodes 233 are respectively mounted in electrode through-holes 2330 provided at the bottom of the base part 23.

Referring to FIGs.2, and 17-19, the base part 23 is provided with an air through-hole 235 for communicating with the air chamber 230. The air through-hole 235 is in air communication with the air channel of the air flow sensor arranged in the battery part. The air flow sensor may be activated when air flows in the air channel of the air flow sensor. The air flow sensor may be triggered to power on the electronic vaporizing device to operate. The air through-hole 235 is defined on both sides of the base part 23. Herein, the upper portion of the air through-hole 235 is defined by a tubular part 2351 and protrudes from the inner bottom surface of the base part. An inclined cover 2352 provided with an air inlet aperture 2353 is placed on the top of the tubular part 2351.

Referring to FIGs.2, 4, and 17, the outer surface of the base part 23 is formed at two ends with blind vias 2340, in which magnets or magnetic materials 234 may be embedded. The magnets or magnetic materials 234 and another set of magnets or magnetic materials (not shown in the drawings) provided on the battery part may have a mutual magnetic attraction force for magnetically connecting the vaporizer with the battery part. In such a case, it is convenient to assemble and disassemble the vaporizer and the battery part. Furthermore, due to constant magnetic attraction force, the vaporizer can keep in close contact with the electrodes of the battery part to ensure stable electrical connection when the vaporizer and the battery part are connected together.

The working principle of the embodiment is as follows.

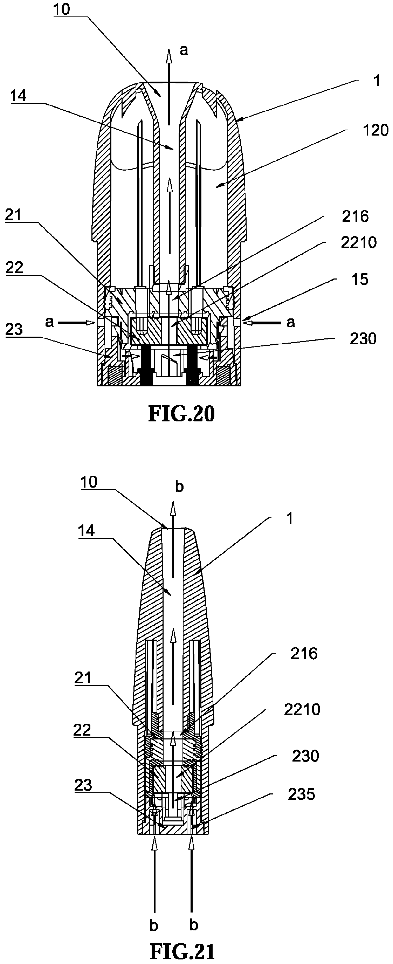

Referring to FIG.20, when a user applies a suction force through the mouthpiece 10, a negative pressure may be generated inside the vaporizer. Outside air may enter through the main air inlet 15 and flow to the air chamber 230 through the gap between the vaporizing core base 21 and the base part 23 in a direction indicated by the arrow a in the drawings. Meanwhile, the to-be-vaporized liquid stored in the liquid storage chamber 120 may flow downwards to the liquid guiding groove 2211 of the vaporizing core through the liquid through-hole 217. Then, the to-be-vaporized liquid may permeate through the vaporizing core body 221 and spread from the liquid guiding groove 2211 to the heating resistance 222 placed at the bottom surface of the vaporizing core body 221. Then, the to-be-vaporized liquid may be heated by means of the heating resistance 222 and vaporized into steam or vapor fog in the air chamber 230. Then, the produced steam or vapor fog may flow through the vaporizing passage 2210, the vapor fog through-hole 216, the vapor outlet tube 14, the mouthpiece 10 and then may be inhaled by the user.

Referring to FIG.21, as mentioned above, the negative pressure may be generated inside the vaporizer, for example inside the air chamber 230, when a user applies a suction force through the mouthpiece 10. In such a case, ail may flow upwards through the air through-hole 235 of the base part, in a direction indicated by the arrow b in the drawings. As the air through-hole 235 is in communication with the air channel of the air flow sensor arranged in the battery part, ail may further flow through the air channel of the air flow sensor, such that the air flow sensor may be activated to power on the electronic vaporizing device to work.

### Other embodiments

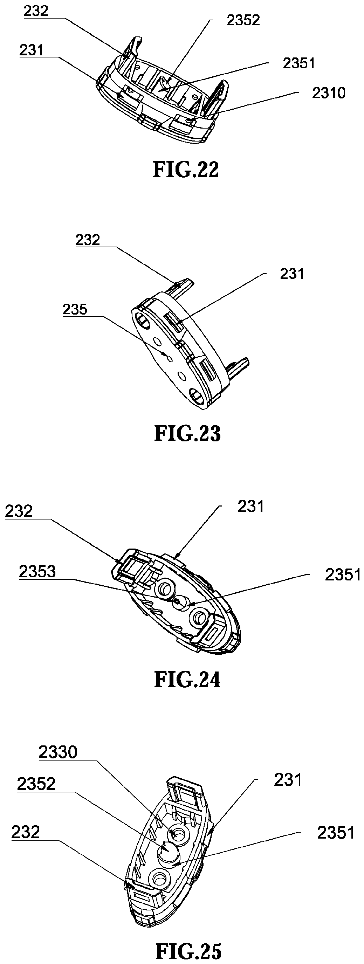

In yet another embodiment, in contrast to the previous embodiments, the main air inlet 15 may be integrated with the first engaging holes 121 (not shown in the drawings). In such a case, the base part 23 may have a different structure, as shown in FIG.22, the first snap bumps 231 of the base part 23 may be provided with second main air inlets 2310 in communication with the first engaging holes 121. Accordingly, outside air may enter the air chamber 230 through the first engaging holes 121 and the second main air inlets 2310.

In still another embodiment, in contrast to the previous embodiments, the base part 23 may have a different structure, as shown in FIGs.23 and 24, the base part 23 is centrally formed with an air through-hole 235 in communication with the air chamber 230. The upper portion of the air through-hole 235 is defined by the tubular part 2351 and protrudes from the inner bottom surface of the base part. The top portion of the tubular part 2351 is provided with an air inlet aperture 2353.

In still another embodiment, in contrast to the previous embodiments, the base part 23 may have a different structure, as shown in FIGs.23-27, the base part 23 is centrally formed with an air through-hole 235 in communication with the air chamber 230. The upper portion of the air through-hole 235 is defined by the tubular part 2351 and protrudes from the inner bottom surface of the base part. The inclined cover 2352 is placed on the top of the tubular part 2351. One end of the inclined cover 2352 is placed higher than the wall portion of the tubular part 2351 to expose an air entrance 2354.

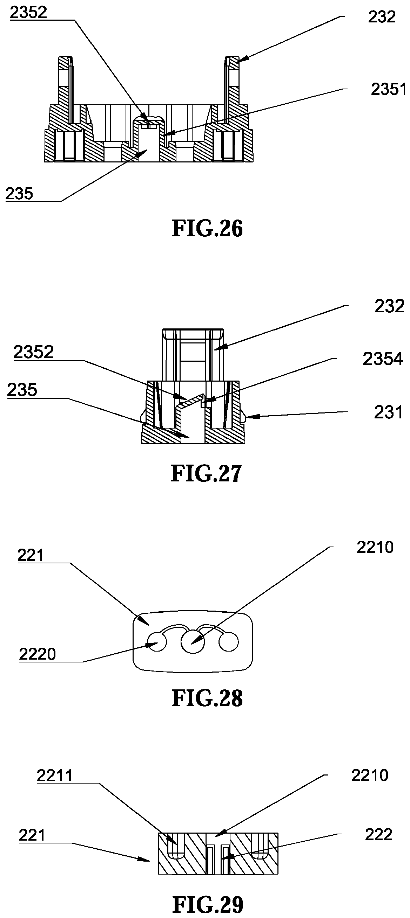

In still another embodiment, in contrast to the previous embodiments, the vaporizing core 22 may have a different structure, as shown in FIGs.28 and 29, the heating resistance 222 is provided on the inner wall of the central through-hole 2210 of the vaporizing core body 221, with two ends of the heating resistance 222 being connected with the planar contact terminals 2220. The planar contact terminals 2220 are likewise disposed on the lower surface of the vaporizing core body 221.

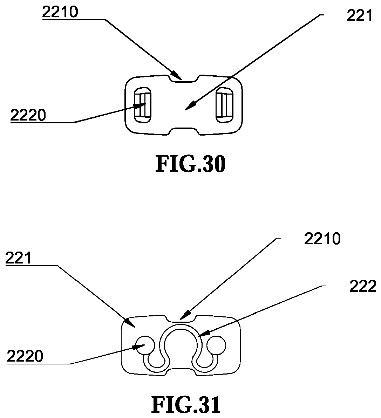

In still another embodiment, in contrast to the previous embodiments, the vaporizing core 22 may have a different structure, as shown in FIGs.30 and 31, the vaporizing passage 2210 of the vaporizing core body 221 is defined by vertical grooves 2210 provided on the two sides of the vaporizing core body.

In further embodiments, any combination of the various structures of the base part as described above and the various structures of the vaporizing core as described above may be provided.

### Industrial applicability

All the above are merely preferred embodiments of the disclosure. The present invention is intended to cover all modifications and equivalent arrangements those skilled in the art can make according to the technical essence of the present invention.

## Claims

1. A vaporizer having an integrated vaporizing assembly, comprising a housing, the housing has a mouthpiece end and a connecting end, the mouthpiece end is provided with a mouthpiece, the connecting end is provided with an opening, the housing is configured to be connected with a battery part by the connecting end, to form an electronic vaporizing device, **characterized in that**, an integrated vaporizing assembly is further provided, the integrated vaporizing assembly is capable of being placed into the housing via the opening of the connecting end, wherein the integrated vaporizing assembly is provided with, from top to bottom, a vaporizing core base, a vaporizing core, and a base part connected with the vaporizing core base, a lower portion of the vaporizing core base is provided with a cavity which is concave upward, the vaporizing core is mounted in the cavity, the vaporizing core comprises a vaporizing core body for transmission of to-be-vaporized liquid and a heating resistance for heating the to-be-vaporized liquid, the vaporizing core body is provided with a vaporizing passage, an air chamber in communication with the vaporizing passage is further provided between the vaporizing core and the base part, and the base part closes the opening of the housing.

2. The vaporizer having an integrated vaporizing assembly according to claim 1, **characterized in that**, a vapor fog through-hole which is in communication with the mouthpiece and the vaporizing passage is provided inside the vaporizing core base.

3. The vaporizer having an integrated vaporizing assembly according to claim 2, **characterized in that**, the vapor fog through-hole is centrally formed in the vaporizing core base.

4. The vaporizer having an integrated vaporizing assembly according to claim 1, **characterized in that**, a space between the vaporizing core base and an inner wall of the housing defines a liquid storage chamber for storing the to-be-vaporized liquid, and the vaporizing core base is internally provided with a liquid through-hole for communicating the liquid storage chamber and the vaporizing core.

5. The vaporizer having an integrated vaporizing assembly according to claim 4, **characterized in that**, an upper portion of vaporizing core body is provided with a liquid guiding groove which is concave downward and in communication with the liquid through-hole.

6. The vaporizer having an integrated vaporizing assembly according to claim 1, **characterized in that**, the vaporizing passage of the vaporizing core body is defined by the central through-hole centrally formed in the vaporizing core body or by vertical grooves provided on two sides of the vaporizing core body.

7. The vaporizer having an integrated vaporizing assembly according to claim 1, **characterized in that**, the mouthpiece extends inwards and is integrally formed with a vapor outlet tube, the vaporizing core base comprises an upper section, a middle section, and a lower section, which have different outer diameters, wherein the upper section of the vaporizing core base is formed as a tube-like connector for connecting with a lower end of the vapor outlet tube, an outer wall of the middle section of the vaporizing core base is sheathed in an inner wall of the housing, and an outer wall of the lower section of the vaporizing core base is connected to an inner wall of an upper portion of the base part.

8. The vaporizer having an integrated vaporizing assembly according to claim 1, **characterized in that**, the vaporizing core base is formed by a support portion and a sealing portion which are embedded with each other, wherein the sealing portion comprises a tube-like connector, an outer wall of the middle section of the vaporizing core base, and an inner wall of the cavity of the vaporizing core base, the support portion is made of hard material, and the sealing portion is made of soft material.

9. The vaporizer having an integrated vaporizing assembly according to claim 1, **characterized in that**, first engaging holes are respectively provided on two sides of the connecting end, and a side wall of the base part is provided with first snap bumps for respectively engaging with the first engaging holes.

10. The vaporizer having an integrated vaporizing assembly according to claim 1, **characterized in that**, an outer wall of the lower portion of the vaporizing core base is provided with second snap bumps, two ends of the base part are arranged with protruding columns, respectively, and the protruding columns are provided with second engaging holes for respectively engaging with the second snap bumps.

11. The vaporizer having an integrated vaporizing assembly according to claim 1, **characterized in that**, a supporting part for propping up the vaporizing core body is provided inside the base part.

12. The vaporizer having an integrated vaporizing assembly according to claim 11, **characterized in that**, the supporting part includes two positive and negative electrode columns served as positive and negative electrodes, and the positive and negative electrode columns are respectively connected with two ends of the heating resistance through contact.

13. The vaporizer having an integrated vaporizing assembly according to claim 1, **characterized in that**, a wall portion of the connecting end is provided with a main air inlet in communication with the air chamber.

14. The vaporizer having an integrated vaporizing assembly according to claim 1, **characterized in that**, the base part is provided with an air through-hole in communication with the air chamber.

15. The vaporizer having an integrated vaporizing assembly according to claim 14, **characterized in that**, the air through-hole is centrally formed in the base part or is provided on two sides of the base part, and an upper portion of the air through-hole is defined by a tubular part and protrudes from an inner bottom surface of the base part.

16. The vaporizer having an integrated vaporizing assembly according to claim 15, **characterized in that**, an inclined cover is placed on a top of the tubular part, wherein the inclined cover is provided with an air inlet aperture, or one end of the inclined cover is placed higher than a wall portion of the tubular part to define an air entrance.

17. The vaporizer having an integrated vaporizing assembly according to claim 1, **characterized in that**, the vaporizing core body is made of microporous ceramic material or microporous diatomaceous material.

18. The vaporizer having an integrated vaporizing assembly according to claim 1, **characterized in that**, the heating resistance is arranged on a lower surface of the vaporizing core body or on a surface of the vaporizing passage.

19. The vaporizer having an integrated vaporizing assembly according to claim 1, **characterized in that**, the heating resistance comprises two planar contact terminals respectively electrically connected with positive and negative electrode columns of the base part through contact.

20. The vaporizer having an integrated vaporizing assembly according to claim 1, **characterized in that**, an outer surface of the base part is embedded with a magnet or a magnetic material, and the magnet or magnetic material and another set of magnet or magnetic material provided on the battery part have a mutual magnetic attraction force for magnetically connecting the vaporizer with the battery part.
